# EUROPEAN PATENT APPLICATION

(11) **EP 1 958 616 A1**
(43) Date of publication of application: **20.08.2008**
(21) Application number: 07090017.0
(22) Date of filing: 13.02.2007
(51) Int. Cl.: A61K 9/16, A61K 31/502

(54) **Pharmaceutical formulations of 1-[4-chloroanilino]-4-[4-pyridylmethyl] phthalazine or salts thereof**

(71) Applicant: Bayer Schering Pharma Aktiengesellschaft, 13353 Berlin (DE)
(72) Inventor: Kranz, Heiko, 10715 Berlin (DE); Jürgens, Kai, 13505 Berlin (DE); Gysler, Jens, 14059 Berlin (DE); Wagner, Torsten, 13127 Berlin (DE)

(57) **Abstract**

The present invention relates to solid pharmaceutical formulations comprising 1-[4-chloroanilino]-4-[4-pyridylmethyl]phthalazine, or a pharmaceutically acceptable salt thereof, to solid dosage forms comprising said solid pharmaceutical formulations, to methods of preparing said solid pharmaceutical formulations and said solid dosage forms, to uses of said solid pharmaceutical formulations, alone or in combination with one or more pharmaceutically active compounds, for the manufacture of a medicament for the treatment especially of a proliferative disease, such as cancer, and to a method of treatment of a proliferative disease, such as cancer.

## Description

### FIELD OF THE INVENTION

The present invention relates to solid pharmaceutical formulations comprising 1-[4-chloroanilino]-4-[4-pyridylmethyl]phthalazine, or a pharmaceutically acceptable salt thereof, to solid dosage forms comprising said solid pharmaceutical formulations, to methods of preparing said solid pharmaceutical formulations and said solid dosage forms, to uses of said solid pharmaceutical formulations, alone or in combination with one or more pharmaceutically active compounds, for the manufacture of a medicament for the treatment especially of a proliferative disease, such as cancer, and to a method of treatment of a proliferative disease, such as cancer.

### BACKGROUND OF THE INVENTION

Two processes, namely the *de novo* formation of vessels from differentiating endothelial cells or angioblasts in the developing embryo (vasculogenesis) and the growth of new capillary vessels from existing blood vessels (angiogenesis), are involved in the development of the vascular systems of animal organs and tissues. Transient phases of new vessel formation (neovascularisation) also occur in the adult body, for example during the menstrual cycle, during pregnancy or during wound healing.

However, a number of diseases are known to be associated with deregulated angiogenesis, for example retinopathies, psoriasis, haemangioblastoma, haemangioma, and neoplastic diseases (solid tumours). Furthermore, the complex processes of vasculogenesis and angiogenesis have been found to involve a whole range of molecules, especially angiogenic growth factors and their endothelial receptors, as well as cell adhesion molecules.

Recent findings have shown that during embryonic development, during normal growth and in a wide number of pathological conditions and diseases, the angiogenic factor known as "Vascular Endothelial Growth Factor" (or "VEGF") forms part of the network regulating the growth and differentiation of the vascular system and its components (G. Breier et al., Trends in Cell Biology, 1996, 6, 454-456 and references cited therein).

VEGF, or more specifically VEGF-A, is a dimeric, disulphide-linked 46 kDa glycoprotein and is structurally related to "Platelet-Derived Growth Factor" (or "PDGF"). It is produced by normal cell lines and tumour cell lines. VEGF is an endothelial cell-specific mitogen and shows angiogenic activity in *in vivo* test systems (e.g. rabbit cornea). VEGF is chemotactic for endothelial cells and monocytes, and induces plasminogen activators in endothelial cells, which are then involved in the proteolytic degradation of the extracellular matrix during formation of capillaries. A number of splice variants of VEGF-A are known which show comparable biological activity, but which differ in the type of cells that secrete them and in their heparin-binding capacity. In addition, there are other members of the VEGF family, such as "Placental Growth Factor" (or "PLGF"), VEGF-B, VEGF-C and VEGF-D.

A large number of human tumours, especially gliomas and carcinomas, express high levels of the VEGF variants and their receptors. This has led to the hypothesis that the VEGF released by tumour cells could stimulate the growth of blood capillaries and the proliferation of tumour endothelium in a paracrine manner and thus, through the improved blood supply, accelerates tumour growth. Increased VEGF expression could explain the occurrence of cerebral oedema in patients with glioma. Direct evidence of the role of VEGF as a tumour angiogenesis factor *in vivo* has been obtained from studies in which VEGF expression or VEGF activity was inhibited. This was achieved with antibodies which inhibit VEGF activity, with dominant-negative VEGFR-2 mutants which inhibited signal transduction, as well as with use of antisense-VEGF RNA techniques. All approaches led to a reduction in the growth of glioma cell lines or other tumour cell lines *in vivo* as a result of inhibited tumour angiogenesis.

There are three VEGFR receptors with different affinities to the ligands. VEGF-A binds to VEGFR1 and VEGFR2 ; VEGF-B and Placental Growth Factor bind to VEGFR1 ; VEGF-A and processed forms of VEGF-C and VEGF-D bind to VEGFR-2 ; VEGF-C and VEGF-D bind to VEGFR-3.

VEGFR-3 is especially important for the growth of lymphatic vessels which play a role in tumour and metastases formation. Also in another diseases state, asthma, the lymphatic tissue is of major importance as it does remain in the alveoli after an acute inflammation and does not resolve like the blood vessels. This leads to the continuing susceptibility to stimulation by foreign agents.

All these receptors are transmembrane proteins. The signal of the VEGF variants is transmitted via the dimerisation of two receptor molecules inducing thereby an activation of the enzymatic activity at the intracellular C-terminal end. The enzymatic activity is a signal kinase, which transfers phosphates from ATP to itself (autophosphorylation) and to downstream signal molecules. This allows for interaction with an entire intracellular signal cascade eventually leading to endothelial cell proliferation and migration. The macroscopic result is the formation of new vessels.

WO 98/35958 describes generically a series of phthalazine derivatives with angiogenesis inhibiting activity and specifically 1-[4-chloroanilino]-4-[4-pyridylmethyl]phthalazine, including salts thereof, in particular the succinic acid salt thereof, as an interesting candidate for treatment of tumours. This compound is an inhibitor of all three VEGFR kinases. The inhibition is not dependent on a specific ligand, but blocks all the signals.

1-[4-chloroanilino]-4-[4-pyridylmethyl]phthalazine has the chemical structure below :

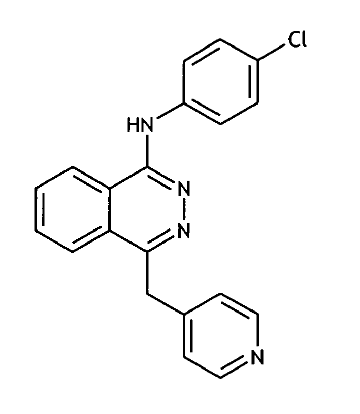

1-[4-chloroanilino]-4-[4-pyridylmethyl]phthalazine is also known as (4-chlorophenyl)[4-(4-pyridylmethyl)-phthalazin-1-yl], or ZK 226343. 1-[4-chloroanilino]-4-[4-pyridylmethyl]phthalazine succinate is also known as PTK/ZK , PTK 787, CGP-79787D, or ZK 222584.

The solubility of PTK/ZK is extremely dependent on pH. PTK/ZK has a reasonable solubility at very low pH values, whereas the solubility decreases significantly as the pH is increased :

| pH | Buffered | Solubility (mg/ml) | |
|---|---|---|---|
| | | 37°C | 20°C |
| 1.0 | no | 108 | |
| 1.1 | yes | | 83 |
| 2.0 | no | 146 | |
| 3.0 | yes | 7.9 | |
| 3.1 | yes | | 7.2 |
| 3.6 | no | 0.35 | |
| 3.7 | no | | 0.34 |
| 4.5 | yes | 0.02 | |
| 5.0 | yes | 3.7 x 10⁻³ | 2.9 x 10⁻³ |
| 7.0 | yes | 7.1 x 10⁻⁴ | 3.1 x 10⁻⁴ |

Since PTK/ZK is absorbed in the small intestine, where the pH is usually above 5, it is of utmost importance that essentially all of the PTK/ZK is dissolved before entering the small intestine, *i.e.* essentially all of the PTK/ZK should be dissolved in the gastric juice. Clearly, in order to obtain satisfactory absorption of the PTK/ZK, the PTK/ZK must be administered in an immediate-release formulation.

Hence, there is a clear need for an easy-to-swallow dosage form combining a high-drug load with a fast, preferably immediate release of the drug.

Very surprisingly, the solution to this problem has been unexpectedly solved by the present inventors upon the discovery that a formulation of the drug which is combined with a matrix former and a pore former and which is prepared as a pellet, surprisingly provides an immediate-release and high-load solid pharmaceutical formulation comprising 1-[4-chloroanilino]-4-[4-pyridylmethyl]phthalazine, or a pharmaceutically acceptable salt thereof, which is very convenient for the patient in that it is very easy to swallow. It was furthermore very surprisingly found that it is possible to store said formulation over time. This added technical advantage thus makes said formulation indeed suitable for clinical supply and commercialisation.

Thus, the object of the present invention is to provide an immediate-release and high-load solid pharmaceutical formulation of 1-[4-chloroanilino]-4-[4-pyridylmethyl]phthalazine, or a pharmaceutically acceptable salt thereof, which exhibits an immediate release of the drug and which is very convenient for the patient in that it is very easy to swallow, and which can be stored over periods of time. This object is met by the solid pharmaceutical formulations described in the present description and defined in the claims appended hereto.

### SUMMARY OF THE PRESENT INVENTION

In a first aspect, the present invention provides a solid pharmaceutical formulation, which comprises :
a) 1-[4-chloroanilino]-4-[4-pyridylmethyl]phthalazine, or a pharmaceutically acceptable salt thereof ;
b) a matrix former (b) ; and
c) a pore former (c) ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI basket method or rotating paddle method using phosphate buffer pH 3.0 at 37°C as the dissolution media and 100 rpm stirring rate,
with the proviso that, said matrix former (b) and said pore former (c) are not identical.

In a preferred embodiment, the present invention provides a solid pharmaceutical formulation in which at least 75%, preferably at least 80%, of said 1-[4-chloroanilino]-4-[4-pyridylmethyl]phthalazine, or pharmaceutically acceptable salt thereof, is released from said solid pharmaceutical formulation within 30 minutes.

In an embodiment, the present invention provides a solid pharmaceutical formulation in which least one pharmaceutically-acceptable excipient is additionally present. Preferably, said pharmaceutically-acceptable excipient is a filler (d).

In an embodiment, in said solid pharmaceutical formulation, said matrix former (b) is a micro-crystalline cellulose-containing matrix former.

In another embodiment, in said formulation, said pore former (c) is a poly(ethylene glycol)-containing pore former. Preferably, said poly(ethylene glycol)-containing pore former (c) is poly(ethylene glycol) 6000.

In another embodiment, in said solid pharmaceutical formulation, said filler (d) is a saccharide-containing filler. Preferably, said saccharide-containing filler is selected from the group comprising, preferably consisting of, lactose, maltodextrin, sucrose, and mannitol. More preferably, said saccharide-containing filler is mannitol.

In an embodiment, in said solid pharmaceutical formulation, said poly(ethylene glycol)-containing pore former (c) is present in an amount of around 0.1 to 10%, preferably around 5%, by weight of the total weight of said solid pharmaceutical formulation.

In another embodiment, in said solid pharmaceutical formulation, said micro-crystalline cellulose-containing matrix former (b) is present in an amount of around 0.1 to 30%, preferably 1 to 20%, more preferably 1 to 5%, particularly around 5%, by weight of the total weight of said solid pharmaceutical formulation.

In another embodiment, in said solid pharmaceutical formulation, said 1-[4-chloroanilino]-4-[4-pyridylmethyl]phthalazine, or pharmaceutically acceptable salt thereof, is present in an amount of around 80 to 90%, preferably around 82.5 to 87.5%, more preferably 87.3%, by weight of the total weight of said solid pharmaceutical formulation.

In another embodiment, in said solid pharmaceutical formulation, said filler (d) is present in an amount of around 0 to 5%, preferably around 2.5 to 3%, more preferably around 2.7%, of the total weight of said solid pharmaceutical formulation.

In a further embodiment, in said solid pharmaceutical formulation, said 1-[4-chloroanilino]-4-[4-pyridylmethyl]phthalazine is in the form of a pharmaceutically acceptable salt thereof. Preferably, said pharmaceutically acceptable salt thereof is an acid addition salt. Notably, said pharmaceutically acceptable salt of 1-[4-chloroanilino]-4-[4-pyridylmethyl]phthalazine is a succinate.

It is to be understood that none of constituents a), b), c), or d) can be the same.

In a second aspect, the present invention provides a solid dosage form comprising a solid pharmaceutical formulation as defined *supra.*

In an embodiment, said solid dosage form *supra* is in a solid unit dosage form. Preferably, said solid unit dosage form is adapted for oral administration.

In an embodiment, said solid unit dosage form is a single solid unit dosage form or a multiple solid unit dosage form.

Particularly, said single solid unit dosage form is in the form of a tablet or a capsule, particularly a hard gelatin capsule, preferably a tablet. In a further embodiment, said tablet is coated. Preferably, said tablet is film-coated.

Particularly, said multiple solid unit dosage form is in pellet form. In a further embodiment, said pellet is coated. Preferably, said coated pellet is film-coated. Alternatively, said multiple solid unit dosage form is present in a capsule, particularly a hard gelatin capsule. Particularly, said capsule containing the multiple solid unit dosage form above is coated, preferably film-coated.

In an embodiment, said multiple solid unit dosage form is present in a sachet, in particular a stick pack or is in the form of an aqueous dispersion.

In a third aspect, the present invention provides a method of preparing a solid pharmaceutical formulation comprising a) 1-[4-chloroanilino]-4-[4-pyridylmethyl]phthalazine, or a pharmaceutically acceptable salt thereof, b) a micro-crystalline cellulose-containing matrix former, and c) a poly(ethylene glycol)-containing pore former, said method comprising the steps of :
a) blending a mixture of said 1-[4-chloroanilino]-4-[4-pyridylmethyl]phthalazine, or a pharmaceutically acceptable salt thereof, said micro-crystalline cellulose-containing matrix former, and said poly(ethylene glycol)-containing pore former, thereby forming a blended mixture ;
b) adding water to said blended mixture thereby forming a granulate;
c) extruding, or spheronising, said granulate thereby forming pellets, particularly round pellets ;
d) drying said pellets, thereby forming dried pellets ; and
e) screening said dried pellets, thereby forming core pellets.

In a fourth aspect, the present invention provides a solid pharmaceutical formulation obtainable by the method according to the third aspect.

In a fifth aspect, the present invention provides a method of preparing a solid unit dosage form according to the third aspect of the invention, said method comprising the steps of :
i) preparing a solid pharmaceutical formulation as in the third aspect for example, in the form of core pellets ; and
ii) formulating said solid pharmaceutical formulation in the form of core pellets into a solid unit dosage form.

In an embodiment, said method further comprises coating said core pellets with a coating agent, preferably a coating suspension, or coating dispersion, thus providing a film-coated pellet.

In a sixth aspect, the present invention provides a solid unit dosage form obtainable by the method according to the fifth aspect of the invention.

In a seventh aspect, the present invention provides a solid pharmaceutical formulation according to the present invention, or as preparable by a method according to the present invention, or a solid dosage form according to the present invention, or as preparable according to a method according to the present invention, for use as a medicament.

In an eighth aspect, the present invention provides the use of a solid pharmaceutical formulation according to the present invention or as preparable by a method according to the invention, or a solid dosage form according to the present invention, or as preparable according to a method of the present invention, for the manufacture of a medicament for the treatment of a proliferative disease, particularly cancer.

In an embodiment, said use can be in combination with a chemotherapeutic agent.

In a ninth aspect, the present invention provides a method of treating a proliferative disease, particularly cancer, said method comprising administering a therapeutically effective amount of the solid pharmaceutical formulation according to the present invention, or as preparable according to a method of the present invention, or a solid dosage form according to the present invention, or as preparable according to a method of the present invention, to a patient in need thereof.

In an embodiment, said method comprises administering a therapeutically effective amount of the solid pharmaceutical formulation according to the invention, or as preparable according to a method according to the invention, or a solid dosage form according to the present invention, or as preparable according to a method according to the present invention, and a chemotherapeutic agent, to a patient in need thereof.

It is to be understood that the present invention covers all possible combinations of the embodiments described in this text.

### BRIEF DESCRIPTION OF THE DRAWINGS

The invention is better understood and other objects, features and advantages of the invention are clearly shown from the following description which refers to the appended Figures. These are given solely as non-limiting Examples which illustrate the invention.

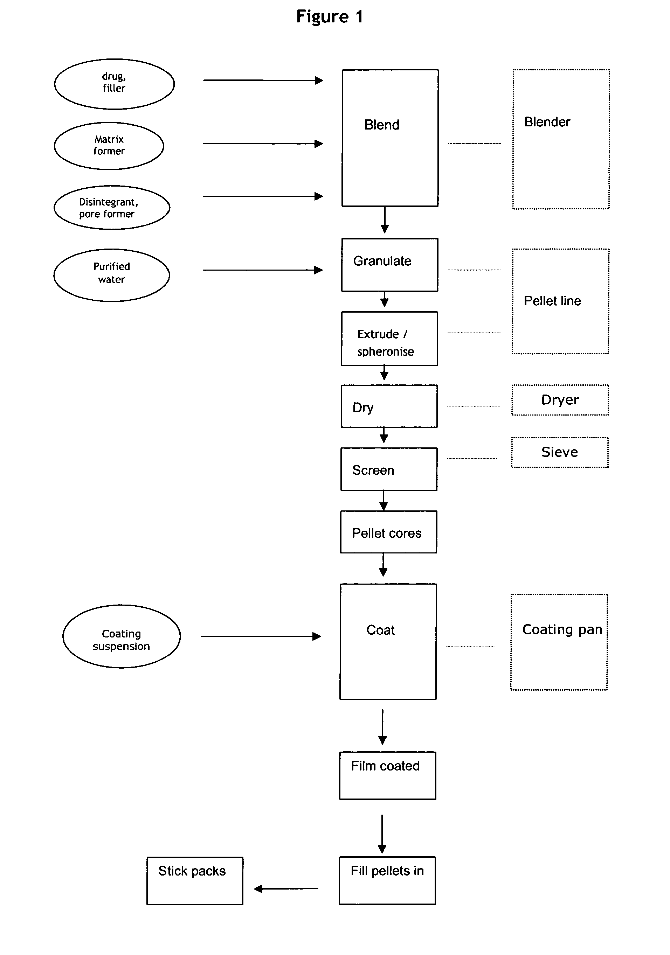

Figure 1 shows a Flow Chart illustrating a manufacturing process according to the present invention of the solid pharmaceutical formulation, and of the solid dosage form containing said solid pharmaceutical formulation, according to the present invention.
Figure 2 shows the results of a set of experiments on the effect of various *in vitro* release methods on the release of 1-[4-chloroanilino]-4-[4-pyridylmethyl]phthalazine (Formulation Example 5).
Figure 3 shows the results of a set of experiments on the effect of the addition of increasing amounts of Ac-Di-Sol (a disintegrant) on the release of 1-[4-chloroanilino]-4-[4-pyridylmethyl]phthalazine (Formulation Examples 2, 6, 7, 11 and 14).
Figure 4 shows the results of a set of experiments on the effect of different types of filter (d) on the release of 1-[4-chloroanilino]-4-[4-pyridylmethyl]phthalazine (Formulation Examples 16, 18, 19 and 20).
Figure 5 shows the results of a set of experiments on the effect of pellet size on the release of 1-[4-chloroanilino]-4-[4-pyridylmethyl]phthalazine from Formulation Example 11.
Figure 6 shows the results of a set of experiments on the effect of fast dissolving pellet coat (hydroxypropyl methyl cellulose) on the release of 1-[4-chloroanilino]-4-[4-pyridylmethyl]phthalazine from Formulation Example 17.
Figure 7 shows the release profiles of 1-[4-chloroanilino]-4-[4-pyridylmethyl]phthalazine from pellets containing Formulation Example 21, placed on stability for one month.
Figure 8 shows the release profiles of 1-[4-chloroanilino]-4-[4-pyridylmethyl]phthalazine from pellets containing Formulation Example 22 (containing MCC/PEG/mannitol), after storage for 3 months.

### DETAILED DESCRIPTION OF THE INVENTION

As mentioned *supra,* the present invention relates to solid pharmaceutical formulations, which comprise :
a) 1-[4-chloroanilino]-4-[4-pyridylmethyl]phthalazine, or a pharmaceutically acceptable salt thereof ;
b) a matrix former (b) ; and
c) a pore former (c) ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI basket method or rotating paddle method using phosphate buffer pH 3.0 at 37°C as the dissolution media and 100 rpm stirring rate,
with the proviso that, said matrix former (b) and said pore former (c) are not identical.

### With reference to the drug PTK/ZK :

As mentioned above, the drug itself may be the free base, *i.e.* 1-[4-chloroanilino]-4-[4-pyridylmethyl]phthalazine, or, indeed, a pharmaceutically acceptable salt thereof, in particular an acid addition salt. Such salts may be formed from suitable inorganic or organic acids. Examples of suitable inorganic acids include the halogen acids, such as hydrochloric acid ; sulphuric acid ; and phosphoric acid. Examples of suitable organic acids include phosphonic, sulphonic or sulphamic acids, carboxylic acids, such as acetic acid, propionic acid, octanoic acid, decanoic acid, dodecanoic acid, glycolic acid, lactic acid, 2-hydroxybutyric acid, gluconic acid, glucose monocarboxylic acid, fumaric acid, succinic acid, adipic acid, pimelic acid, suberic acid, azelaic acid, malic acid, tartaric acid, citric acid, glucaric acid, galactaric acid ; amino acids, such as glutamic acid, aspartic acid, N-methylglycine, acetylaminoacetic acid, N-acetylasparagine and N-acetylcysteine ; pyruvic acid, acetoacetic acid, phosphoserine, 2- or 3-glycerophosphoric acid, glucose-6-phosphoric acid, glucose-1-phosphoric acid, fructose-1,6-bis-phosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, mandelic acid, cinnamic acid, glucuronic acid, galacturonic acid, methane- or ethanesulphonic acid, 2-hydroxyethanesulphonic acid, ethane-1,2-disulphonic acid, 2-, 3-, or 4-methylbenzenesulphonic acid, methylsulphuric acid, ethylsulphuric acid, dodecylsulphuric acid, N-cyclohexylsulphamic acid, N-methyl-, N-ethyl-, or N-propyl-sutphamic acid, or other organic acids, such as ascorbic acid.

As mentioned above, the term "PTK/ZK" refers to the succinate salt of 1-[4-chloroanilino]-4-[4-pyridylmethyl]phthalazine. PTK/ZK is described in WO 98/35958.

### In re. the matrix former (b) :

The term "matrix former" is understood as meaning a pharmaceutically-inert ingredient included in the formulation in order to procure a matrix which provides physical stability, *i.e.* mechanical stability, e.g. binding stability, of the dosage form. Particularly, said matrix former can be cellulose, for example a starch-containing compound, for example cellulose or starch, particularly micro-crystalline cellulose (or "MCC"), for example Avicel PH 101 (marketed by FMC BioPolymer), such as sucrose, glucose, sorbitol, acacia, alginic acid, sodium alginate, gelatin, starch, pregelatinised starch, magnesium aluminium silicate, carboxymethylcellulose sodium (or "CMC sodium"), methylcellulose, ethylcellulose, hydroxypropyl methylcellulose (or "HPMC"), polyvinylacetate, polyethylene glycol or polyvinylpyrrolidone (or "PVP"), e.g. PVP K12, PVP K15, PVP K17, PVP K25, PVP K30, PVP K60, PVP K90, PVP K120, carrageenan, such as Gelcarin GP 812 (marketed by FMC BioPolymer) or any combination thereof.

### In re. the pore former (c) :

The pore former (c) in accordance with the present invention is a readily soluble excipient which is present in the formulation and allows pores to be introduced into the solid pharmaceutical formulation, thus allowing the solid pharmaceutical formulation to disintegrate rapidly. The pore former is a pore former which is well-known to the person skilled in the art of drug formulation, notably poly(ethylene glycol) (or "PEG"), particularly a PEG having a molecular weight of 4,000 to 10,000 Daltons, (or "PEG 4000" or "PEG 10000", respectively), preferably a PEG having a molecular weight of 6,000 (or "PEG 6000"). polyvinylpyrrolidone (or "PVP"), a saccharide, e.g. a monosaccharide, such as dextrose, mannose, fructose, a disaccharide, such as sucrose, an oligosaccharide, such as sucrose or glucodifructose.

It is reiterated here that in the context of the present invention, said matrix former (b) and said pore former (c) are not identical.

As explained *supra,* the present invention surprisingly provides an immediate-release and high-load solid pharmaceutical formulation comprising 1-[4-chloroanilino]-4-[4-pyridylmethyl]phthalazine, or a pharmaceutically acceptable salt thereof, which is combined with a matrix former and a pore former and which is prepared as a pellet, and which provides an immediate release dosage form which is very convenient for the patient in that it is very easy to swallow. Further, as mentioned *supra,* it is possible to store said formulation over time. This added technical advantage thus makes said formulation indeed suitable for clinical supply and commercialisation.

### Solid pharmaceutical formulation

As will be understood from the present application, including the Examples provided herein, it is of utmost importance that the 1-[4-chloroanilino]-4-[4-pyridylmethyl]phthalazine, or a pharmaceutically acceptable salt thereof, is released in a fast and reliable manner under acidic conditions. Thus, in the present context, the terms "fast-release" or "immediate-release" mean that at least 70% of the 1-[4-chloroanilino]-4-[4-pyridylmethyl]phthalazine, or a pharmaceutically acceptable salt thereof, is dissolved from the solid pharmaceutical formulation within 30 minutes as determined by the USP XXVI basket method or rotating paddle method using phosphate buffer pH 3.0, particularly 0.05 M KH₂PO₄/HCl buffer, at 37°C as the dissolution media and 100 rpm stirring rate. In a preferred embodiment of the invention at least 75%, more preferably at least 80%, of the 1-[4-chloroanilino]-4-[4-pyridylmethyl]phthalazine, or a pharmaceutically acceptable salt thereof, is dissolved from the solid pharmaceutical formulation within 30 minutes as determined by the USP XXVI basket method or rotating paddle method described herein.

As will also be understood from the present disclosure it is of utmost importance that the solid pharmaceutical formulation contains a "high-load" of 1-[4-chloroanilino]-4-[4-pyridylmethyl]phthalazine, or a pharmaceutically acceptable salt thereof, due to the relative large amount of 1-[4-chloroanilino]-4-[4-pyridylmethyl]phthalazine, or a pharmaceutically acceptable salt thereof, needed for administration to patients. Thus, in the present context, the terms "high-load" or "high-drug-load" mean that the solid pharmaceutical formulation contains at least 50% by weight, calculated on the basis of the total weight of the formulation, of the 1-[4-chloroanilino]-4-[4-pyridylmethyl]phthalazine, or a pharmaceutically acceptable salt thereof. In a particular embodiment of the present invention, the solid pharmaceutical formulation comprises at least 55% by weight, at least 60% by weight, at least 65% by weight, at least 70% by weight, at least 75% by weight, at least 80% by weight, at least 85% by weight, or at least 90% by weight, of 1-[4-chloroanilino]-4-[4-pyridylmethyl]phthalazine, or a pharmaceutically acceptable salt thereof, calculated on the basis of the total weight of the formulation. Stated otherwise, the solid pharmaceutical formulation typically comprises 50-90% by weight, calculated on the basis of the total weight of the formulation, of 1-[4-chloroanilino]-4-[4-pyridylmethyl]phthalazine, or a pharmaceutically acceptable salt thereof.

Particularly, said solid pharmaceutical formulation according to the present invention contains 1-[4-chloroanilino]-4-[4-pyridylmethyl]phthalazine, or pharmaceutically acceptable salt thereof, in an amount of around 80-90%, preferably around 82.5 to 87.5%, more preferably 87.3%, by weight of the total weight of said solid pharmaceutical formulation.

As mentioned *supra,* the solid pharmaceutical formulation may, in addition to the 1-[4-chloroanilino]-4-[4-pyridylmethyl]phthalazine, or a pharmaceutically acceptable salt thereof, the matrix former (b), and the pore former (c), *supra,* contain one or more additional pharmaceutically acceptable excipients.

In particular, said at least one pharmaceutically acceptable excipient is a filler (d).

### Filler (d) :

Notably, said filler (d) is known to the person skilled in the art and can be, for example :
- an inert diluent or filler, such as a saccharide-containing filler, such as sucrose, maltodextrin, sorbitol, a sugar, mannitol, micro-crystalline cellulose, a starch, sodium chloride, sodium phosphate, calcium carbonate, calcium phosphate, calcium sulphate or lactose, e.g. lactose monohydrate.

In a preferred embodiment of the invention, the inert filler or diluent is mannitol.

In accordance with the present invention, said solid pharmaceutical formulation contains said filler (d) in an amount of around 0 to 5%, preferably around 2.5 to 3%, more preferably around 2.7%, of the total weight of said solid pharmaceutical formulation.
- Lubricants, including glidants and antiadhesives,
   such as magnesium stearate, zinc stearate, stearic acid, silicas, hydrogenated vegetable oils or talc. The lubricant is typically present in an amount from 0.1-10% by weight of the total formulation. Preferably, the lubricant is present in an amount from 0.2-5% by weight of the total formulation, such as from 0.5-5% by weight of the total formulation, e.g. from 1-5% by weight of the total formulation, more preferably in an amount from 1-3% by weight of the total formulation. In a preferred embodiment of the invention, the lubricant is magnesium stearate.
- Disintegrants,
   such as a carboxymethylcellulose-based disintegrant, such as croscarmellose sodium, such as Ac-Di-Sol (marketed by FMC BioPolymer), cross-linked povidone, sodium starch glycolate, maize starch or potato starch.
- Surfactants and wetting agents,
   such as naturally occurring phosphatides, e.g. lechitin or soybean lechitin ; condensation products of ethylene oxide with e.g. a fatty acid, a long chain fatty alcohol, or a partial ester derived from fatty acids and a hexitol or a hexitol anhydride, for example polyoxyethylene stearate, polyoxyethylene sorbitol monooleate, polyoxyethylene sorbitan monooleate, etc. ; or salts of long-chain aliphatic phosphates, such as sodium lauryl sulphate.

It is to be understood that none of constituents a), b), c), or d) can be the same.

Examples of other pharmaceutically acceptable excipients which may be incorporated in the solid pharmaceutical formulation of the invention include colorants, flavouring agents, plasticizers, humectants, buffering agents, etc.

The solid pharmaceutical formulations of the invention may also contain further drug substances, such as anti-cancer agents.

In those cases where the pharmaceutical formulation is in the form of a solid dosage form, in particular a solid unit dosage form (e.g. a tablet, a sachet, in particular a stick pack, or capsule, particularly a hard gelatin capsule, in particular a tablet), the unit dosage form is adapted for oral administration and may be provided with a coating, such as a film coating, a sugar coating, or the like.

Thus, a suitable coating for the solid unit dosage form according to the invention may, for example, be a sugar coating or a film coating based on one or more of the ingredients : hydroxypropyl methylcellulose (or "HPMC"), methylcellulose, ethylcellulose, hydroxyethyl methylcellulose, hydroxypropylcellulose, carboxymethylcellulose sodium, acrylate polymers (e.g. Eudragit^{®} (marketed by Degussa), polyethylene glycols or polyvinylpyrrolidone, or a colorant, such as talc, titanium dioxide, a ferrous pigment. Preferably, the coating is a film coating based on HPMC.

### Medical use

The present invention provides means and methods for treating certain cancers or tumours or tumour angiogenesis, in any suitable animal, preferably in a mammal, and in particular in a human being.

The term "solid malignant tumour" is understood as meaning understood as meaning an abnormal malignant mass of tissue that is not inflammatory, which arises without obvious cause from cells of preexistent tissue, which possesses no physiologic function and which has the ability to invade normal cells and spread throughout the body. Examples of typical solid malignant tumours include breast carcinoma, non-small cell lung cancer, colon carcinoma, renal cell carcinoma and malignant melanoma.

The term "carcinoma" is understood as meaning understood as meaning a malignant tumour of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term ""sarcoma" is understood as meaning understood as meaning a malignant tumour growing from connective tissue, such as cartilage, fat, muscles, tendons and bones.

The term "leukaemia" is understood as meaning a blood cancer, *i*.*e*. a cancer that originates from the bone marrow and which keeps the marrow from producing normal red and white blood cells and platelets.

The term "lymphoma" refers to a cancer that originates in the nodes or glands of the lymphatic system.

The term "glioma", when used herein, is intended to cover a malignant tumour originating from glial cells.

The term "inhibition"" or "inhibit" as used in connection with treatment of solid tumours is intended to cover the delayed appearance of primary or secondary tumours, slowed development of primary or secondary tumours, decreased occurrence of primary or secondary tumours, slowed or decreased severity of secondary effects of disease, arrested tumour growth and regression of tumours. The term "reduction" or "reducing" in connection with tumour size is covered by the term "inhibition" or "inhibit". The reduction of the solid tumour refers to a reduction of the tumour volume. For example, a 10% reduction of a solid tumour means that the volume of the treated tumour has been reduced by 10%.

An important aspect of the present invention lies in the therapeutic application of the solid pharmaceutical formulation of the invention.

Thus, the present invention is also directed to a solid pharmaceutical formulation of the invention for use as a medicament. In particular, the present invention is directed to use of the solid pharmaceutical formulation of the invention for the manufacture of a medicament for treatment of cancer. Stated otherwise, the present invention is directed to a method of treating cancer, said method comprising administering a therapeutically effective amount of the solid pharmaceutical formulation of the invention to a patient in need thereof.

In the present context, the term "treatment of a cancer" or "treating a mammal having a cancer" is understood as meaning that the solid pharmaceutical formulation described herein is administered in a therapeutically effective amount which is sufficient to *i*) inhibit growth of the tumour, *ii)* facilitate tumour regression, *i.e.* reduce the size of the tumour, *iii)* remove the tumour and/or *iv)* inhibit cancer cell metastasis.

The solid pharmaceutical formulation will be administered to patients in a "therapeutically effective" dose, *i.e.* in a dose that is sufficient to produce the desired effects in relation to the condition for which it is administered. The exact dose will depend on the cancer form to be treated, and will be ascertainable by one skilled in the art using known techniques. A suitable dose of the PTK/ZK, is contemplated to be in the range of about 0.5-3 g/patient/day, preferably 1-2 g/patient/day, such as 1-1.5 g/patient/day, e.g. 1.3-1.4 g/patient/day, in particular 1.34 g/patient/day.

In a very interesting embodiment of the invention said cancer is a carcinoma, such as a carcinoma selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumours, in particular selected from the group consisting of malignant melanoma, non-small cell lung cancer, breast carcinoma, colon carcinoma and renal cell carcinoma.

Thus, in one embodiment of the invention said cancer is malignant melanoma, such as superficial spreading melanoma, nodular melanoma, lentigo maligna melanoma, acral melagnoma, amelanotic melanoma or desmoplastic melanoma. In another embodiment of the invention said cancer is non-small cell lung cancer. In still another embodiment of the invention said cancer is breast carcinoma. In a further embodiment of the invention said cancer is colon carcinoma. In an even further embodiment of the invention said cancer is renal cell carcinoma.

In a further interesting embodiment of the invention said cancer is a sarcoma, such as a sarcoma selected from the group consisting of osteosarcoma, Ewing's sarcoma, chondrosarcoma, malignant fibrous histiocytoma, fibrosarcoma and Kaposi's sarcoma.

In an even further interesting embodiment of the invention said cancer is a glioma.

As will be understood by the skilled person, the above-mentioned cancer types (*i*.*e*. carcinomas, sarcomas and gliomas) are characterised by the presence of solid tumours.

In an even further interesting embodiment of the invention said cancer is a lymphoma, such as a lymphoma selected from the group consisting of Hodgkin's disease, non-Hodgkin's disease, B-cell lymphoma, T-cell lymphoma, follicular lymphoma, Burkitt's lymphoma and mycosis fungoides.

In still another interesting embodiment of the invention said cancer is a myeloma, such as multiple myeloma.

One important use in cancer therapy is the reduction and blockade of ascites formation in abdominal tumours of different origin (e.g. uterine endometrium, ovarial cancer, colon cancer) also for mesothelioma.

In addition to its use as anticancer agent the formulation of the invention can be used for other diseases characterised by overshooting angiogenesis, such as macular degeneration due to vessel in-growth, corneal transplantation due to lymphatic vessel in growth and thereby breakage of the immune privilege. It is helpful in diseases such as rheumatoid arthritis with vessels growing ectopically towards the joints. As mentioned above, it is effective in asthma. Also diseases with female cycle associated vessel growth are influenced beneficially as is the case for endometriosis.

It will be understood that an even more effective treatment of the various cancer forms may be obtained by combination therapy where the solid pharmaceutical formulation of the invention is combined with a suitable chemotherapeutic agent and/or is combined with radiotherapy and/or is combined with surgery.

Thus, a further aspect the present invention relates to the use of a solid pharmaceutical formulation of the invention for the manufacture of a medicament for treatment of cancer in combination with a chemotherapeutic agent. Analogously, a further aspect of the present invention relates to a method of treating cancer, said method comprising administering a therapeutically effective amount of the solid pharmaceutical formulation of the invention and a chemotherapeutic agent to a patient in need thereof.

Specific examples of suitable chemotherapeutic agents include chemotherapeutic agents selected from the group consisting of adrenocorticosteroids, such as prednisone, dexamethasone or decadron ; altretamine (hexalen, hexamethylmelamine (HMM)) ; amifostine (ethyol) ; aminoglutethimide (cytadren) ; amsacrine (M-AMSA) ; anastrozole (arimidex) ; androgens, such as testosterone ; asparaginase (elspar); bacillus calmette-gurin ; bicalutamide (casodex) ; bleomycin (blenoxane) ; busulphan (myleran) ; carboplatin (paraplatin) ; carmustine (BCNU, BiCNU) ; chlorambucil (leukeran) ; chlorodeoxyadenosine (2-CDA, cladribine, leustatin) ; cisplatin (platinol) ; cytosine arabinoside (cytarabine) ; dacarbazine (DTIC) ; dactinomycin (actinomycin-D, cosmegen) ; daunorubicin (cerubidine) ; docetaxel (taxotere) ; doxorubicin (adriomycin) ; epirubicin ; estramustine (emcyt) ; estrogens, such as diethylstilbestrol (DES) ; etopside (VP-16, VePesid, etopophos) ; fludarabine (fludara) ; flutamide (eulexin) ; 5-FUDR (floxuridine) ; 5-fluorouracil (5-FU) ; gemcitabine (gemzar) ; goserelin (zodatex) ; herceptin (trastuzumab) ; hydroxyurea (hydrea) ; idarubicin (idamycin) ; ifosfamide ; IL-2 (proleukin, aldesteukin) ; interferon alpha (intron A, roferon A) ; irinotecan (camptosar) ; leuprolide (lupron) ; levamisole (ergamisole) ; lomustine (CCNU) ; mechlorathamine (mustargen, nitrogen mustard) ; melphalan (alkeran) ; mercaptopurine (purinethol, 6-MP) ; methotrexate (mexate) ; mitomycin-C (mutamucin) ; mitoxantrone (novantrone) ; octreotide (sandostatin) ; pentostatin (2-deoxycoformycin, nipent) ; plicamycin (mithramycin, mithracin) ; prorocarbazine (matulane) ; streptozocin ; tamoxifin (nolvadex) ; taxol (paclitaxel) ; teniposide (vumon, VM-26) ; thiotepa ; topotecan (hycamtin) ; tretinoin (vesanoid, all-trans retinoic acid) ; vinblastine (valban) ; vincristine (oncovin) and vinorelbine (navelbine).

Other chemotherapeutic agents, which may be useful for the purposes described herein include the chemotherapeutic agents mentioned in column 12, line 62 to column 13, line 42 of US 6,482,802, which is herein incorporated by reference. For a description of these and other chemotherapeutic agents, see The Merck Index, 12th edition, pp. THER 13-14, which is herein incorporated by reference.

It will be understood that the chemotherapeutic agent is selected under due consideration of the actual cancer form. In a preferred embodiment of the invention the following chemotherapeutic agents are used (together with the solid pharmaceutical formulation described herein) for treatment of the following specific cancer forms : e.g. malignant melanoma and cisplatin ; malignant melanoma and IL-2 ; renal cell carcinoma and doxorubicin ; renal cell carcinoma and IL-2 ; breast carcinoma and doxorubicin ; breast carcinoma and taxol ; colon carcinoma and 5-fluorouracil ; colon carcinoma and cisplatin ; and non-small cell lung cancer and cisplatin.

The invention is further described in the following Examples. The Examples are not, in any manner, to be understood as limiting the generality of the present specification and claims.

### EXAMPLES

For all the formulations the first step of the manufacturing process is a mixing step that is followed by an extrusion/spheronisation step. Afterwards, the obtained pellets are optionally coated with an immediate release film coat. The film coated pellets are then optionally filled into stick packs. The details for the different formulations are described below.

### Description of the formulations

The components of the different formulations serve for the following purposes :

**Table 1: Functionality of the individual ingredients**

| **substance** | **Function** |
|---|---|
| **Vatalanib succinate** | active ingredient |
| **micro-crystalline cellulose, « MCC » Avicel PH 101** | matrix former |
| **carrageenan** | matrix former |
| **Croscarmellose Na, Ac-Di-Sol** | Disintegrant |
| **PEG 6000** | pore former |
| **Lactose monohydrate** | Filler |
| **Mannitol** | Filler |
| **Sucrose** | Filler |
| **Maltodextrin** | Filler |
| **Hydroxypropyl methylcellulose** | film coating, binder |
| **Talc** | film coating, anti sticking agent |
| **Titanium dioxide** | film coating, colorant |
| **Ferrous pigment** | film coating, colorant |
| **Purified water** | processing aid for extrusion and film coating |

The different formulations consist of the components listed below. All excipients are well known and routinely used in oral drug products.

### EXAMPLE 1 : PREPARATION OF A SOLID PHARMACEUTICAL FORMULATION ACCORDING TO THE INVENTION

Vatalanib succinate (873.0g (87.3% by weight)) ("PTK/ZK"), Avicel PH 101 (50.0g ((5.0% by weight)) as matrix former (b), PEG 6000 (50.0g ((5.0% by weight)) as pore former (c), and mannitol (27.0g ((2.7% by weight)) as filler (d) are placed in a blender, are mixed and are blended until uniform. The blended mixture is fed into a granulator in a pellet line and is granulated by the addition of water. The granulate is then extruded and spheronised to form pellets. The pellets are then dried in a fluid bed drier at 50° C, until the humidity of the pellets is equal to the relative humidity of the surrounding air, to provide dried pellets. Afterwards the pellets were optionally stored for 24 hours at ambient conditions. The dried pellets are then screened by passing them through suitable screens to exclude small and large fractions. Suitable pellets for the invention may be pellets in the size range of 0.71 mm - 1.25 mm.

By analogy with the method described in Example 1, the solid pharmaceutical formulations of Formulation Examples 2 to 27 were prepared. The composition of said Examples are given in Table 2, *infra :*

**Table 2: Composition of the investigated pellet formulations :**

| Ex am pl e | PTK/ZK % by weight" | Matrix former % by weight** | Disintegrant % by weight" | Pore former % by weight** | Filler % by weight" | Water content* % by weight** |
|---|---|---|---|---|---|---|
| 2 | **90.0** | Avicel PH 101 **10.0** | --- | --- | --- | --- |
| 3 | **90.0** | Avicel PH 101 **10.0** | --- | --- | --- | **35.0** |
| 4 | **90.0** | Avicel PH 101 **5.0** | Ac-Di-Sol **5.0** | --- | --- | **60.0** |
| 5 | **100.0** | --- | --- | --- | --- | **15.0** |
| 6 | **90.0** | Avicel PH 101 **3.0** | Ac-Di-Sol **5.0** | PEG 6000 **2.0** | --- | **45.0** |
| 7 | **90.0** | Avicel PH 101 **9.0** | Ac-Di-Sol **1.0** | --- | --- | **40.0** |
| 8 | **90.0** | Avicel PH 101 **7.5** | Ac-Di-Sol **2.5** | --- | --- | **45.7** |
| 9 | **90.0** | Avicel PH 101 **6.0** | Ac-Di-Sol **4.0** | --- | --- | **55.0** |
| 10 | **90.0** | Avicel PH 101 **6.0** | Ac-Di-Sol **4.0** | --- | --- | **57.5** |
| 11 | **87.3** | Avicel PH 101 **5.0** | Ac-Di-Sol **5.0** | --- | Lactose **2.7** | **60.0** |
| 12 | **87.3** | Avicel PH 101 **5.0** | Ac-Di-Sol **5.0** | --- | Lactose **2.7** | **60.0** |
| 13 | **87.3** | Avicel PH 101 **5.0** | Ac-Di-Sol **5.0** | --- | Lactose **2.7** | **60.0** |
| 14 | **87.3** | Avicel PH 101 **5.0** | Ac-Di-Sol **5.0** | --- | Lactose **2.7** | **60.0** |
| 15 | **87.3** | Avicel PH 101 **6.0** | Ac-Di-Sol **4.0** | --- | Lactose **2.7** | |
| 16 | **87.3** | Avicet PH 101 **7.5** | Ac-Di-Sol **2.5** | --- | Lactose **2.7** | |
| 17 | **87.3** | Avicel PH 101 **5.0** | Ac-Di-Sol **5.0** | --- | Lactose **2.7** | **60.0** |
| 18 | **87.3** | Avicel PH 101 **6.0** | Ac-Di-Sol **4.0** | --- | Mannitol **2.7** | **61.0** |
| 19 | **87.3** | Avicel PH 101 **5.0** | Ac-Di-Sol **5.0** | --- | Sucrose **2.7** | **62.0** |
| 20 | **87.3** | Avicel PH 101 **5.0** | Ac-Di-Sol **5.0** | --- | Maltodextrin **2.7** | **62.0** |
| 21 | **87.3** | Avicel PH 101 **5.0** | Ac-Di-Sol **5.0** | --- | Mannitol **2.7** | **61.0** |
| 22 | **87.3** | Avicel PH 101 **5.0** | Ac-Di-Sol **5.0** | --- | Mannitol **2.7** | **62.0** |
| 23 | **87.3** | Avicel PH 101 **5.0** | PEG 6000 **5.0** | --- | Mannitol **2.7** | **13.0** |
| 24 | **87.3** | Carrageenan (Gelcarin GP 812) **10.0** | --- | --- | Mannitol **2.7** | **45.0** |
| 25 | **87.3** | Carrageenan (Gelcarin GP 911) **10.0** | --- | --- | Mannitol **2.7** | **40.0** |
| 26 | **87.3** | Carrageenan (Gelcarin GP 911) **5.0** | --- | PEG 6000 **5.0** | Mannitol **2.7** | **15.0** |
| 27 | **87.3** | Avicel PH 101 **5.0** | Ac-Di-Sol **5.0** | --- | Mannitol **2.7** | |

| | | | | | | |
|---|---|---|---|---|---|---|
| *Water content : for granulation, calculated on the basis of total weight of the solid blended mixture ** : the following formulations were all prepared on a 1 kg scale. Hence, e.g. 1% by weight correspond to 10g by weight. | | | | | | |

### EXAMPLE 28 : PREPARATION OF SOLID PHARMACEUTICAL FORMULATION-CONTAINING FILM COATED PELLETS ACCORDING TO THE PRESENT INVENTION

Using solid pharmaceutical formulation Example 18, following extrusion and spheronisation, the core pellets were film-coated with an aqueous dispersion of hydroxypropyl methylcellulose, talc, titanium dioxide and ferrous pigment. Hydroxypropyl methylcellulose is added as binder, talc, titanium dioxide and ferrous pigment are used as colorants. The aqueous coating process is performed to procure protection to the pellets and for optical purposes, *i.e.* to provide a visual aspect for substance identification purposes.

### EXAMPLE 29: PRIMARY PACKAGING OF THE SOLID PHARMACEUTICAL FORMULATIONS OF EXAMPLE 18 AND OF THE SOLID PHARMACEUTICAL FORMULATION-CONTAINING FILM COATED PELLETS OF EXAMPLE 28 ACCORDING TO THE PRESENT INVENTION

After extrusion and spheronisation of the solid pharmaceutical formulation of the invention, and optional coating thereof, the formulations are placed in stick packs, each being filled with 2,000 mg pellets, as primary packaging.

### EXAMPLE 30 : FILLING OF THE SOLID PHARMACEUTICAL FORMULATIONS OF EXAMPLE 18 AND OF THE SOLID PHARMACEUTICAL FORMULATION-CONTAINING FILM COATED PELLETS OF EXAMPLE 28 ACCORDING TO THE PRESENT INVENTION INTO HARD GELATIN CAPSULES

After extrusion and spheronisation of the solid pharmaceutical formulation of the invention, and optional coating thereof, the formulations are placed in hard gelatin capsules, each being filled with 50 mg pellets.

### EXAMPLE 31 : DRUG RELEASE STUDIES

*In vitro* drug release was determined using USP XXVI basket method or rotating paddle method :

| | |
|---|---|
| Apparatus : | USP dissolution apparatus 2 with six covered glass vessels and basket or paddle stirrers, Distek Premiere 5100 Dissolution System, Distek Inc., North Brunswick, USA. |
| Medium : | 0.05 M phosphate buffer (KH₂PO₄/HCl), degassed. The pH value was finally adjusted to 3.00±0.05 |
| Filling volume : | 1000 ml, unless stated otherwise |
| Temperature : | 37°C±0.5°C |
| Stirring rate : | 100 rpm±2 rpm |
| Sampling times : | 10, 15, 30, 45 and 60 minutes |
| Detection : | UV-measurement at 316 nm |

For the rotating pellet method, the pellets were pre-wetted with 100 ml of the dissolution medium in order to avoid floating of the pellets. At predetermined time intervals, 10 ml samples were withdrawn (not replaced), filtered and assayed. The amount of vatalanib succinate released was measured UV-spectrophotometrically at 316 nm (Beckmann photometer DU 640, Beckmann, USA). Stock solutions of vatalanib succinate of known concentration were used to calculate the amount of drug released.

### Example 31A : Effect of different in vitro release methods on the release of Vatalanib succinate ("PTK/ZK") (Formulation Example 6).

Pellets containing up to 90% (w/w, based on the total weight of the core pellets) Vatalanib succinate were successfully prepared by extrusion and spheronisation in accordance with Example 6.

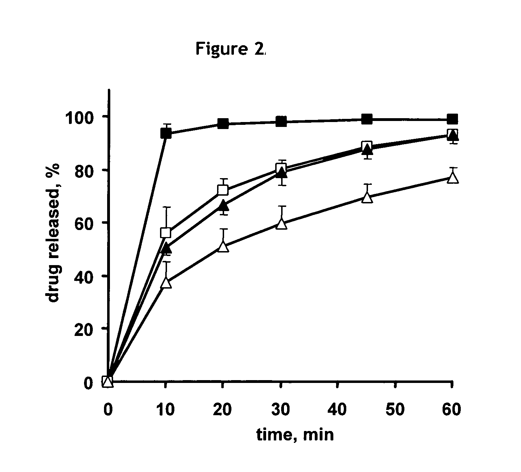

The dissolution profile was obtained according to Example 30, and the results of a set of experiments on different in vitro release methods are shown in Figure 2, wherein :
**-■- paddle 372 mg pellets (1000ml)**
**-□- basket 186 mg pellets (500ml)**
**-▲- basket 186 mg pellets(1000ml)**
**-△- basket 372 mg pellets (1000ml)**

For the basket method it was shown that the *in vitro* release rate of Vatalanib succinate was only independent of the amount of release medium when filling a lower amount of pellets (186 mg) into the baskets. However, *in vitro* drug release studies should be done on a typical single unit dose of active. Therefore, the amount of pellets filled into the baskets was increased to 372 mg. When increasing the amount of pellets within the baskets the drug release dropped drastically even when using the higher amount of release medium. In contrast, for the paddle method fast drug release was observed even when using the higher amount of pellets. As the *in vitro* dissolution method should primarily be independent of the amount of pellets within the dissolution vessel, the paddle method was used for further experiments. 372 mg pellets were filled in each vessel at further dissolution testing as this amount of pellets contains a typical single dose of the active compound. In order to avoid floating of the pellets, pre-wetting of the pellets is essential when using the paddle method.

The aim of the study was to develop an immediate release pellet formulation for Vatalanib succinate. The pellet formulation *should* demonstrate an *in vitro* dissolution profile of at least 75% drug release within 30 min (Q=70%).

### Example 31 B : Effect of the addition of increasing amounts Ac-Di-Sol on the release of Vatalanib succinate.

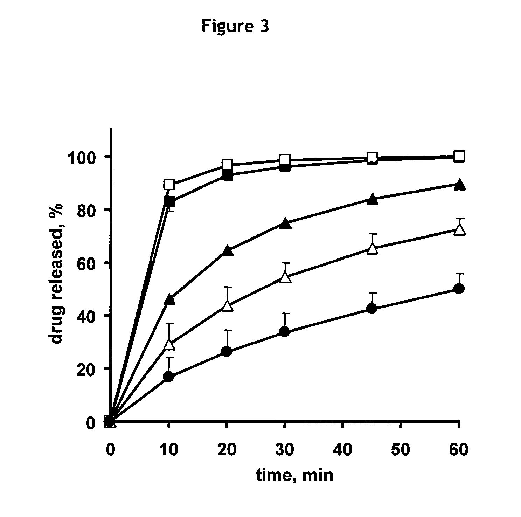

In order to obtain the desired *in vitro* release profile different formulation parameters such as the addition of a disintegrant were investigated : The results are shown in Figure 3, wherein :
**-■- 5% Ac-Di-Sol (Ex. 12)**
**-□- 4% Ac-Di-Sol (Ex. 15)**
**-▲- 2.5% Ac-Di-Sol (Ex. 8)**
**-△- 1% Ac-Di-Sol (Ex. 7)**
**-●- no Ac-Di-Sol (Ex. 3)**

The desired *in vitro* release profile of Q = 70% after 30 min could not be reached when using pellets without the disintegrant Ac-Di-Sol. Therefore, micro-crystalline cellulose was partially replaced by the disintegrant Ac-Di-Sol. It is shown that the desired *in vitro* release profile of Q = 70% after 30 min was reached for formulations containing ≥ 2.5 % (w/w, based on the total pellet mass) Ac-Di-Sol.

In addition, it has to be pointed out that the amount of residual water within the pellet formulations had a significant effect on the *in vitro* drug release profiles. In order to achieve reproducible *in vitro* release profiles for further experiments formulations were dried in a fluid bed dryer at 50°C until rel. humidity of outlet air is constant for 5 min. Afterwards the pellets are stored for 24 hours at ambient conditions.

### Example 31C : Effect of the addition of different type of fillers on the release of Vatalanib succinate.

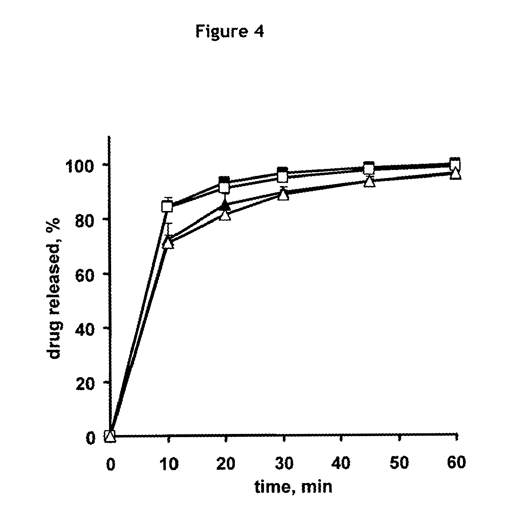

The assay for calculation purposes of different Vatalanib succinate batches can vary (e.g. due to different water contents in the drug). In order to balance this effect a filler is usually added to formulations. Therefore, different type of fillers (lactose, maltodextrin, sucrose, and mannitol) were incorporated into pellets containing 87.3 % active, 5% micro-crystalline cellulose, 5% Ac-Di-Sol and 2.7% filler (%, w/w based on total pellet mass). The pellets were prepared according to Example 1. The effect of the addition of different type of fillers on the *in vitro* release of Vatalanib succinate is given in Figure 4, wherein :
**-■- lactose (Ex. 17)**
**-□- maltodextrine Ex. 20)**
**-▲- sucrose (Ex. 19**)
**-△- mannitol (Ex. 21)**

As shown only slight differences in the *in vitro* release profiles were observed when using different type of fillers. In principle, all the investigated type of fillers can be taken for manufacturing of pellets containing Vatalanib succinate.

### Example 31 D : Effect of the pellet size on the release of Vatalanib succinate (Formulation Example 12).

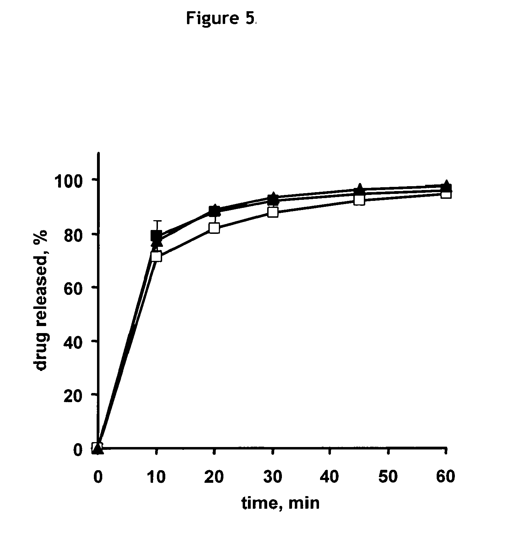

To establish a robust pelletisation process, the release of Vatalanib succinate should be relatively independent of the pellet size. In order to investigate the influence of the pellet size on the *in vitro* drug release, pellets were classified into different pellet size fractions by sieving. Drug release was investigated in phosphate buffer pH 3.0, and the results are shown in Figure 5, wherein :
**-■- 0.71 0.80 mm**
**-□- 0.80 - 1.00 mm**
**-▲- 1.00 - 1.25 mm**

This effect was investigated on formulations containing 87.3 % active, 5% micro-crystalline cellulose, 5% Ac-Di-Sot and 2.7% lactose (%, w/w based on total pellet mass).

For pellets in the size range of 0.71 - 1.25 mm no significant differences in the *in vitro* release profiles were observed. Even when using pellets in the size range of 1.00 mm - 1.25 mm the desired in vitro release profile of Q = 70% was reached. Therefore, it was concluded that pellets in a size range of 0.71 mm - 1.25 mm can be used for further experiments. It has to be noted that this effect was only investigated on formulations containing lactose as filler. However, as the effect of the type of filler on the *in vitro* release profile was negligible it can be assumed that similar results will be obtained for formulations containing mannitol.

### Example 31 E : Effect of a fast dissolving pellet coat (hydroxypropyl methylcellulose) on the release of Vatalanib succinate.

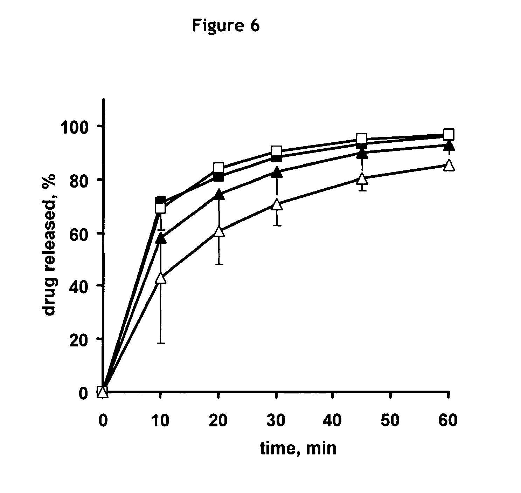

For optical and taste masking purposes pellets were coated with a fast dissolving hydroxypropyl methylcellulose shell. The *in vitro* drug release of uncoated pellets was compared with the release of coated pellets in phosphate buffer pH 3.0, and the results are shown in Figure 6, wherein :
**-■- uncoated**
**-□- 13% c.l.**
**-▲- 23% c.l.**
**-△- 26% c.l.**
(c.l.: coating index, *i.e.* percentage w/w of total solid content based on mass of the core pellets).

This effect was investigated on formulations containing 87.3 % Vatalanib succinate, 6% micro-crystalline cellulose, 4% Ac-Di-SoL and 2.7% lactose (%, w/w based on total pellet mass) (Table 2, Formulation Example 18).

To achieve a uniform coating high amounts of polymer were sprayed onto the core pellets (13% - 26%, w/w total solid content based on mass of the core pellets). After 10 min, 71% drug versus 43% of active compound were released from uncoated pellets and pellets coated with 26% (w/w, based on core pellet mass) hydroxypropyl methylcellulose, respectively. No differences in the dissolution rates were observed at further sampling points. Different dissolution rates for uncoated and coated pellets at the first sampling point can be explained with the additional dissolution step of the coat. Once the fast dissolving pellet shell is dissolved, no differences in dissolution rates were observed. It has to be noted that this effect was only investigated on formulations containing 6% micro-crystalline cellulose, 4% Ac-Di-Sol and 2.7% lactose.

### Example 31 F : Release profiles of Vatalanib succinate from pellets placed on stability for one month.

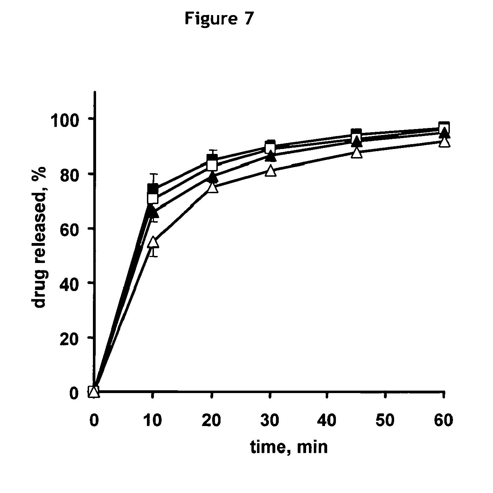

Uncoated pellets were put on stability at 25°C / 60% relative humidity (RH), 30°C / 70% ROH, and 40°C / 75% RH according to the International Committee of Harmonisation (ICH) Guidelines on Stability Studies. After one month of storage, the drug release was investigated in phosphate buffer pH 3.0, and the results are shown in Figure 7, wherein :
**-■-initial**
**-□- 25°C/60% RH**
**-▲- 30°C/70% RH**
**-△- 40°C/75% RH**

This effect was investigated on formulations containing 87.3 % active, 5% micro-crystalline cellulose, 5% Ac-Di-SoL and 2.7% mannitol (%, w/w based on total pellet mass) (Table 2, Formulation Example 22).

The stability studies demonstrated that the drug release rates dropped rapidly. Even after one month the drug release rate dropped from 90% to 81% after 30 min for pellets stored at 40°C/75% RH, (which is considered by the person skilled in the art as accelerated storage conditions). These decreasing release rates are not acceptable and indicate that further formulations improvements are required. It was assumed that the fast decreasing in vitro release rate can be attributed to the presence of the disintegrant Ac-Di-Sol. This excipient may change its properties in the presence of humidity thus leading to decreasing release rates.

### Example 31G : Release profiles of Vatalanib succinate from pellets containing MCC/PEG/mannitol after storage for 3 months.

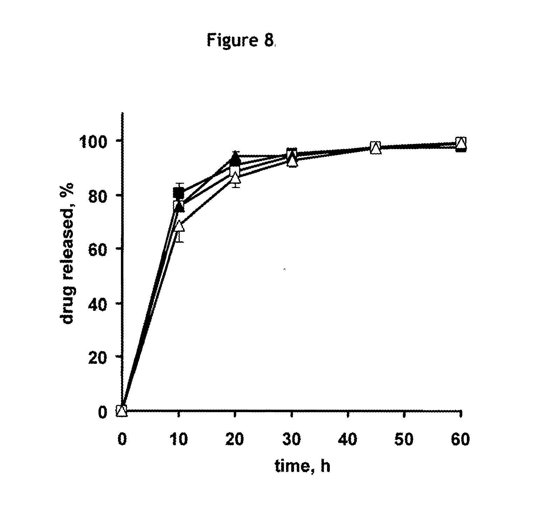

MCC was partially replaced by PEG 6000 (Table 2, Formulation Example 23 : 87.3 % Vatalanib succinate, 5% micro-crystalline cellulose, 5% PEG 6000 and 2.7% mannitol). The initial in vitro drug release and drug release after storage for 3 months was investigated in phosphate buffer pH 3.0, and the results are shown in Figure 8, wherein :
**-■- initial**
**-□- 30°C/70% RH**
**-▲- 25°C/60% RH**
**-**△**- 40°C/75% RH**

As shown in Figure 8, the release of Vatalanib succinate from MCC/PEG/mannitol formulations was fast. Initially 81% and 95% drug was released after 10 and 30 min, respectively. When placing these formulations on stability according to the ICH Guidelines, surprisingly no significant drop in dissolution rate was observed, and even for formulations stored under accelerated conditions, *i.e*. at 40°C/75% RH for 3 months.

In view of the in vitro dissolution profiles being unchanged upon storage, it is clear that this formulation is indeed suitable for clinical supply and commercialisation.

## Claims

1. A solid pharmaceutical formulation, **characterised in that** it comprises :
a) 1-[4-chloroanilino]-4-[4-pyridylmethyl]phthalazine, or a pharmaceutically acceptable salt thereof ;
b) a matrix former (b) ; and
c) a pore former (c) ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI basket method or rotating paddle method using phosphate buffer pH 3.0 at 37°C as the dissolution media and 100 rpm stirring rate,
with the proviso that, said matrix former (b) and said pore former (c) are not identical.

2. The formulation according to claim 1, wherein at least 75%, preferably at least 80%, of said 1-[4-chloroanilino]-4-[4-pyridylmethyl]phthalazine, or pharmaceutically acceptable salt thereof, is released from said solid pharmaceutical formulation within 30 minutes.

3. The formulation according to claim 1 or 2, wherein at least one pharmaceutically-acceptable excipient is additionally present.

4. The formulation according to claim 3, wherein said pharmaceutically-acceptable excipient is a filler (d), with the proviso that none of constituents a), b), c), or d) are the same.

5. The formulation according to any one of claims 1 to 4, wherein said matrix former (b) is a micro-crystalline cellulose-containing matrix former.

6. The formulation according to any one of claims 1 to 5, wherein said pore former (c) is a poly(ethylene glycol)-containing pore former.

7. The formulation according to claim 6, wherein poly(ethylene glycol)-containing pore former (c) is poly(ethylene glycol) 6000.

8. The formulation according to any one of claims 4 to 7, wherein said filler (d) is a saccharide-containing filler.

9. The formulation according to claim 8, wherein said saccharide-containing filler is selected from the group comprising, preferably consisting of, lactose, maltodextrin, sucrose, and mannitol.

10. The formulation according to claim 9, wherein said saccharide-containing filler is mannitol.

11. The formulation according to any one of claims 1 to 10, wherein said poly(ethylene glycol)-containing pore former (c) is present in an amount of around 0.1 to 10%, preferably around 5%, by weight of the total weight of said solid pharmaceutical formulation.

12. The formulation according to any one of claims 1 to 11, wherein said micro-crystalline cellulose-containing matrix former (b) is present in an amount of around 0.1 to 30%, preferably 1 to 20%, more preferably 1 to 5%, particularly around 5%, by weight of the total weight of said solid pharmaceutical formulation.

13. The formulation according to any one of claims 1 to 12, wherein said 1-[4-chloroanilino]-4-[4-pyridylmethyl]phthalazine, or pharmaceutically acceptable salt thereof, is present in an amount of around 80 to 90%, preferably around 82.5 to 87.5%, more preferably 87.3%, by weight of the total weight of said solid pharmaceutical formulation.

14. The formulation according to any one of claims 4 to 13, wherein said filler (d) is present in an amount of around 0 to 5%, preferably around 2.5 to 3%, more preferably around 2.7%, of the total weight of said solid pharmaceutical formulation.

15. The formulation according to any one of claims 1 to 14, wherein said 1-[4-chloroanilino]-4-[4-pyridylmethyl]phthalazine is in the form of a pharmaceutically acceptable salt thereof.

16. The formulation according to claim 15, wherein said pharmaceutically acceptable salt thereof is an acid addition salt.

17. The formulation according to claim 16, wherein said pharmaceutically acceptable salt of 1-[4-chloroanilino]-4-[4-pyridylmethyl]phthalazine is a succinate.

18. A solid dosage form comprising a solid pharmaceutical formulation according to any one of claims 1 to 17.

19. The solid dosage form according to claim 18, which is in a solid unit dosage form.

20. The solid unit dosage form according to claim 19, which is adapted for oral administration.

21. The solid unit dosage form according to claim 19 or 20, which is a single solid unit dosage form or a multiple solid unit dosage form.

22. The single solid unit dosage form according to claim 21, which is in the form of a tablet or a capsule.

23. The single solid unit dosage form according to claim 22, which is in the form of a tablet.

24. The tablet according to claim 23, wherein said tablet is coated.

25. The coated tablet according to claim 24, wherein said tablet is film-coated.

26. The multiple solid unit dosage form according to claim 21, which is in pellet form.

27. The pellet according to claim 26, wherein said pellet is coated.

28. The coated pellet according to claim 27, wherein said coated pellet is film-coated.

29. The multiple solid unit dosage form according to any one of claims 26 to 28, which is present in a capsule, particularly a hard gelatin capsule.

30. The multiple solid unit dosage form according to claim 29, wherein said capsule is coated, preferably film-coated.

31. The multiple solid unit dosage form according to any one of claims 26 to 28, which is present in a sachet, in particular a stick pack.

32. The multiple solid unit dosage form according to any one of claims 26 to 28, which is in the form of an aqueous dispersion.

33. A method of preparing a solid pharmaceutical formulation comprising a) 1-[4-chloroanilino]-4-[4-pyridylmethyl]phthalazine, or a pharmaceutically acceptable salt thereof, b) a micro-crystalline cellulose-containing matrix former, and c) a poly(ethylene glycol)-containing pore former, said method comprising the steps of :
a) blending a mixture of said 1-[4-chloroanilino]-4-[4-pyridylmethyl]phthalazine, or a pharmaceutically acceptable salt thereof, said micro-crystalline cellulose-containing matrix former, and said poly(ethylene glycol)-containing pore former, thereby forming a blended mixture ;
b) adding water to said blended mixture thereby forming a granulate ;
c) extruding, or spheronising, said granulate thereby forming pellets, particularly round pellets ;
d) drying said pellets, thereby forming dried pellets ; and
e) screening said dried pellets, thereby forming core pellets.

34. A solid pharmaceutical formulation obtainable by the method according to claim 33.

35. A method of preparing a solid unit dosage form according to claim 18, said method comprising the steps of :
i) preparing a solid pharmaceutical formulation according to any one of claims 1 to 17, in the form of core pellets ; and
ii) formulating said solid pharmaceutical formulation in the form of core pellets into a solid unit dosage form.

36. The method according to claim 35, which further comprises coating said core pellets with a coating agent, preferably a coating suspension, or coating dispersion, thus providing a film-coated pellet.

37. A solid unit dosage form obtainable by the method according to claim 35 or 36.

38. A solid pharmaceutical formulation according to any of claims 1 to 17, or claim 34, or a solid dosage form according to any of claims 18 to 32, or claim 37, for use as a medicament.

39. Use of a solid pharmaceutical formulation according to any of claims 1 to 17, or claim 34, or a solid dosage form according to any of claims 18 to 32, or claim 37, for the manufacture of a medicament for the treatment of a proliferative disease, particularly cancer.

40. Use of a solid pharmaceutical formulation according to any of claims 1 to 17, or claim 34, or a solid dosage form according to any of claims 18 to 32, or claim 37, for the manufacture of a medicament for the treatment of a proliferative disease, particularly cancer, in combination with a chemotherapeutic agent.

41. A method of treating a proliferative disease, particularly cancer, said method comprising administering a therapeutically effective amount of the solid pharmaceutical formulation according to any of claims 1 to 17, or claim 34, or a solid dosage form according to any of claims 18 to 32, or claim 37, to a patient in need thereof.

42. A method of treating a proliferative disease, particularly cancer, said method comprising administering a therapeutically effective amount of the solid pharmaceutical formulation according to any of claims 1 to 17, or claim 34, or a solid dosage form according to any of claims 18 to 32, or claim 37, and a chemotherapeutic agent to a patient in need thereof.
